# EUROPEAN PATENT APPLICATION

(11) **EP 1 783 232 A1**
(43) Date of publication of application: **09.05.2007**
(21) Application number: 05768811.1
(22) Date of filing: 28.07.2005
(51) Int. Cl.: C12Q 1/68, C12N 15/09, G01N 21/78

(54) **METHOD OF MEASURING HETEROGENOUS NCULEAR NIBONUCLEOPROTEIN B1 (hnRNP B1) mRNA**

(30) Priority: 30.07.2004 JP 2004224098
(71) Applicant: TOSOH CORPORATION, Shunan-shi Yamaguchi-ken 746-8501 (JP)
(72) Inventor: SAITO, Juichi, 2420028 (JP); HAYASHI, Toshinori, 2280804 (JP)
(74) Representative: Pautex Schneider, Nicole Véronique
(86) International application number: PCT/JP2005/014257
(87) International publication number: WO 2006/011667

(57) **Abstract**

A method for assaying heterogeneous nuclear ribonucleoprotein B1 (hnRNP B1) mRNA present in a sample, the method comprising a step of using a first primer homologous to at least a portion downstream from the 5'-end of a specified nucleotide sequence of the RNA and a second primer complementary to at least a portion upstream from the 3'-end of the specified nucleotide sequence to produce double-stranded DNA containing the promoter sequence and the specified nucleotide sequence downstream from the promoter sequence, wherein at least one of the first and second primers has a promoter sequence at the 5'-end, a step of using the double-stranded DNA as template to produce an RNA transcript, a step of using the RNA transcript in turn as template for DNA synthesis to produce the double-stranded DNA, a step of nucleic acid amplification in which the aforementioned steps are repeated under conditions that simultaneously promote each of the steps, and a step of assaying the amount of the RNA transcript.

## Description

### Technical Field

The present invention relates to a method for rapid assay of hnRNP B1 mRNA in a convenient, isothermal and single-stage manner. The invention belongs to the field of medicine and especially clinical diagnosis, and provides a useful index for early diagnosis of cancer, monitoring of treatment, judgment of prognosis and determination of treatment course.

### Background Art

Heterogeneous nuclear ribonucleoprotein (hereinafter, hnRNP) A2/B1 is a major constituent element of hnRNP. hnRNP exists in the nucleus as complexes with heterogeneous nuclear RNA (composed mainly of precursor messenger RNA), and is involved in processing, extranuclear transport and stability of messenger RNA (mRNA). hnRNP A2 and hnRNP B1 are splicing variants, with hnRNP A2 having the same sequence as hnRNP B1 except that 36 nucleotides of the 5'-end of the structural gene being deleted (see Burd, C. G., et al., (1989) Proc. Natl. Acad. Sci. USA, 86, 9788-9792 and Maeda, A., et al., (1994) EMBO J., 13, 5783-5795).

In recent years it has been discovered that hnRNP A2/B1 is over-expressed in pancreatic and lung cancer tissues, and it has attracted interest as a diagnostic marker for cancer (see Japanese Unexamined Patent Publication (Kohyo) No. 2000-500322 and Zhou, J., et al., (1996) J. Biol. Chem., 271, 10760-10766, Fielding, P., et al., (1999) Clin. Cancer Res., 5, 4048-4052, Zhou, J., et al., (2001) Lung Cancer Res., 34, 341-350).

A highly sensitive assay method for measuring the expression hnRNP A2/B1 has been reported, wherein hnRNP A2/B1 mRNA is amplified by RT-PCR and the amount of amplification product is measured (see Japanese Unexamined Patent Publication (Kohyo) No. 2000-500322 and Zhou et al. (2001) op.cit.). Recent research has indicated that hnRNP B1 is over-expressed in human cancer cells from an early stage of cancer. It has been reported that hnRNP B1 mRNA levels increase more specifically than hnRNP A2/B1 in cancer tissue compared to normal tissue, based on assay of hnRNP B1 mRNA by RT-PCR, and that assay of hnRNP B1 expression levels is therefore useful for early diagnosis of lung cancer. (See Sueoka, E., et al., (1999) Cancer Res., 59, 1404-1407, Sueoka, E., et al., (2001) Cancer Res., 61, 1896-1902, Fleischhacker, M., et al., (2001) Ann. N.Y. Acad. Sci., 945, 179-188).

These findings suggest that hnRNP B1 can serve as a more useful cancer marker than hnRNP A2/B1. One means for highly sensitive assay of hnRNP B1 is a method of amplifying hnRNP B1 mRNA by RT-PCR and measuring the amount of amplification product, but this generally requires a two-stage process including a reverse transcription (RT) stage and a PCR stage, which not only complicates the procedure and lowers reproducibility, but also raises the risk of secondary contamination. Also, the RT and PCR stages combined take 2 hours or longer in most cases and the method therefore is unsuitable for processing of multiple specimens or reducing the cost of examinations. Moreover, because DNA is also amplified in RT-PCR, the chromosomal DNA must be completely removed by DNase treatment or the like during the nucleic acid extraction step if the goal is to amplify only mRNA, and this has led to a more complex procedure for nucleic acid extraction. In addition, PCR requires abrupt increase/decrease in the reaction temperature, which has constituted an obstacle against power savings and cost reduction for automated reactors.

On the other hand, methods for amplifying RNA alone at constant temperature have been reported, such as the NASBA method (see Japanese Patent No. 2650159 and Japanese Patent No. 3152927) and the TMA method (see Japanese Patent No. 3241717). These RNA amplification methods employ a chain reaction wherein a primer including the promoter sequence for the target RNA, reverse transcriptase and if necessary Ribonuclease H (RNase H) are used for synthesis of double-stranded DNA containing the promoter sequence, RNA polymerase is used for synthesis of RNA containing the specified nucleotide sequence of the target RNA, and the RNA is in turn used as template for synthesis of double-stranded DNA containing the promoter sequence. After RNA amplification, the amplified RNA is detected by electrophoresis or a hybridization method using a nucleic acid probe bound to a detectable label. These RNA amplification methods are suitable for convenient mRNA assay since they amplify only RNA in an isothermal, single-stage manner, but detection by hybridization methods and the like require complex procedures and do not allow highly reproducible quantitation.

As convenient methods for amplification and assay of mRNA there may be mentioned the method described by Ishiguro et al. (Japanese Unexamined Patent Publication (Kokai) No. 2000-14400 and Ishiguro, T., et al., (2003) Anal. Biochem., 314, 77-86). In this method, RNA amplification is carried out in the presence of a nucleic acid probe which is labeled with an intercalating fluorescent dye and is designed so that when it forms a complementary double strand with the target nucleic acid, the intercalating fluorescent dye moiety undergoes a change in fluorescent property by intercalating into the complementary double strand, and the change in fluorescent property is measured, whereby it is possible to simultaneously accomplish RNA amplification and assay in a convenient, isothermal and single-stage manner in a sealed vessel.

### Disclosure of the Invention

The assay of hnRNP B1 mRNA is useful for early diagnosis of lung cancer and other squamous carcinoma, but using RT-PCR requires a two-stage process with a complicated procedure and necessitating abrupt increase/decrease in the reaction temperature; this leads to a risk of secondary contamination and poor reproducibility, while constituting an obstacle to development of a convenient and automated assay. The present invention overcomes the aforementioned problems by providing a method for assaying hnRNP B1 mRNA in a convenient, rapid, isothermal and single-stage manner.

As a result of much diligent research directed toward solving the aforementioned problems, the present inventors have constructed a method for assaying hnRNP B1 mRNA in a convenient, rapid, isothermal and single-stage manner which applies the RNA amplification method explained above. Specifically, by measuring the level of amplified RNA product obtained by an RNA amplification process wherein a first primer and second primer (at least one of which has a promoter sequence at the 5' end) are used to produce double-stranded DNA containing the promoter sequence, the double-stranded DNA is used as template to produce an RNA transcript, and the RNA transcript in turn is used as template for DNA synthesis to produce the double-stranded DNA, it has become possible to assay hnRNP B1 mRNA in a convenient, isothermal and single-stage manner.

### Brief Explanation of the Drawings

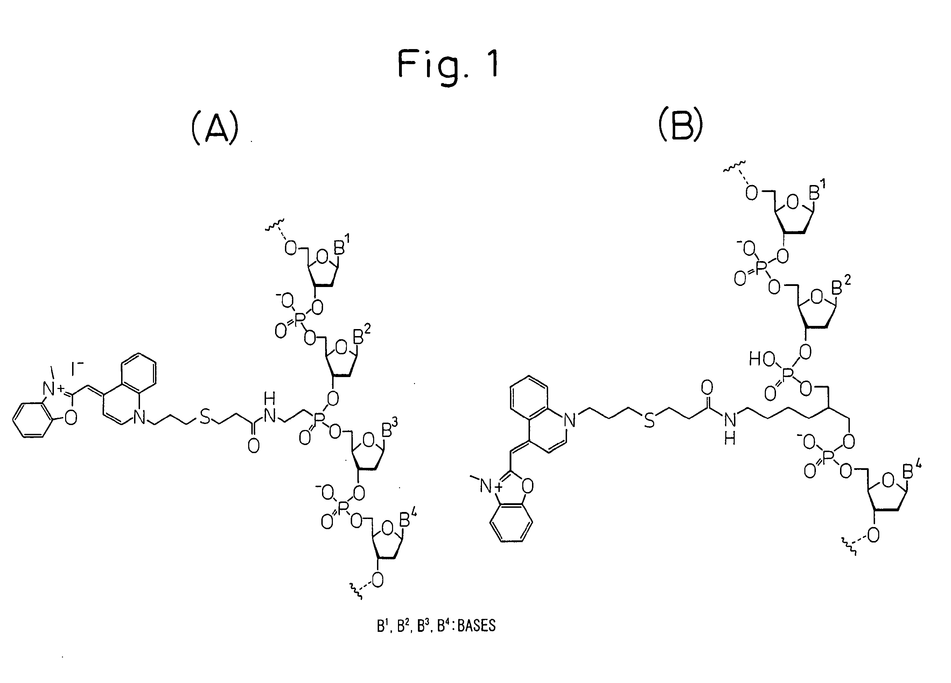

Fig. 1 shows the structure of the intercalating fluorescent dye-labeled nucleic acid probe prepared in Example 2. B¹, B², B³ and B⁴ represent bases.
A) A probe comprising an intercalating fluorescent dye (oxazole yellow) bonded to the phosphate diester moiety via a linker, according to the method of Ishiguro et al. (see Ishiguro, T., et al., (1996) Nucleic Acids Res., 24, 4992-4997). In order to prevent 3'-terminal - OH group extension reaction, the 3'-terminal -OH group was modified with glycolic acid.
B) A probe comprising oxazole yellow bonded according to the method of Ishiguro et al. (see Ishiguro et al. (1996) op.cit.), with an amino group introduced using commercially available Label-ON Reagents (Clontech). Here, the amino group was introduced at the nucleoside-lacking portion at the site of introduction (B³ in the drawing). In order to prevent 3'-terminal -OH group extension reaction, the 3'-terminal -OH group was modified with biotin.

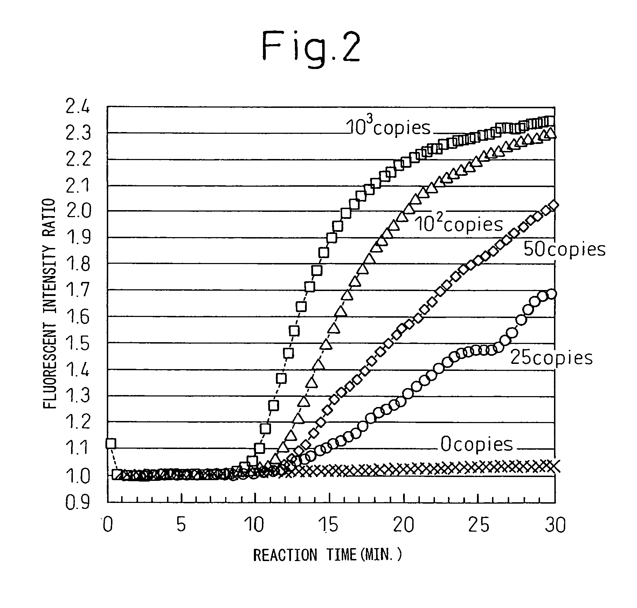

Fig. 2 shows fluorescence profiles obtained as a result of the measurement of Example 3.

Shown are the results of carrying out RNA amplification according to the invention simultaneously with periodic measurement of fluorescent intensity (excitation wavelength: 470 nm, fluorescent wavelength: 520 nm). The horizontal axis represents reaction time, and the vertical axis represents fluorescent intensity ratio (fluorescent intensity of reaction mixture/background fluorescence). The numbers of copies in the figure represent the initial numbers of copies of hnRNP B1 RNA (including base numbers 157-1249) used per test (calculated by absorbance at 260 nm).

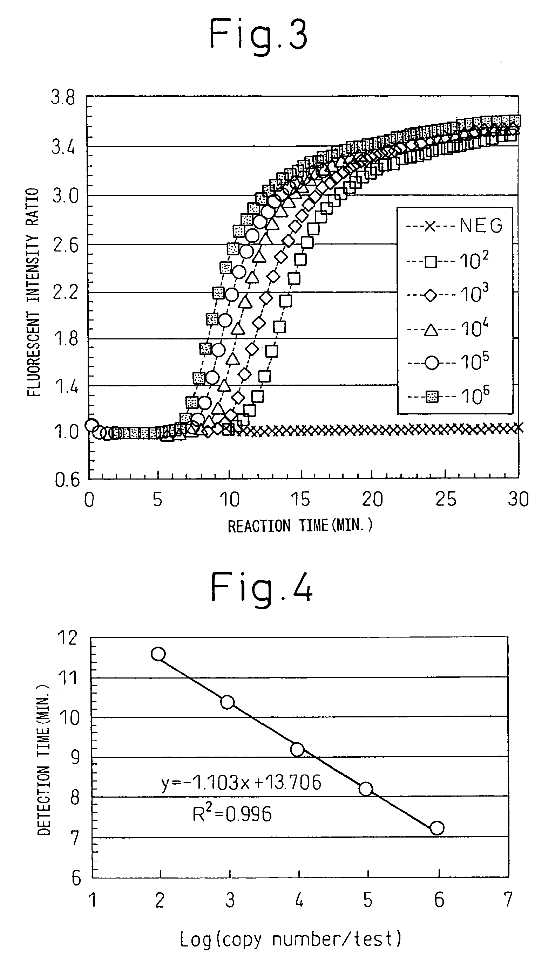

Fig. 3 shows fluorescence profiles obtained as a result of the measurement of Example 4.

Shown are the results of carrying out RNA amplification according to the invention simultaneously with periodic measurement of fluorescent intensity (excitation wavelength: 470 nm, fluorescent wavelength: 520 nm). The horizontal axis represents reaction time, and the vertical axis represents fluorescent intensity ratio (fluorescent intensity of reaction mixture/background fluorescence). The numerals in the legend of the figure represent the initial numbers of copies of hnRNP B1 RNA (including base numbers 157-1249) used per test (calculated by absorbance at 260 nm).

Fig. 4 is a calibration curve obtained from the results of Fig. 3. With the detection time defined as time at which the fluorescent intensity ratio reached 1.2 in the results of Fig. 3, the detection time was plotted with respect to the logarithm of initial number of copies of standard RNA. The initial number of copies of unknown sample is calculated from this calibration curve and the detection time of the unknown sample obtained by the present method.

### Best Mode for Carrying Out the Invention

The present invention will now be explained in detail.

The invention is a method for assaying heterogeneous nuclear ribonucleoprotein B1 (hnRNP B1) mRNA present in a sample, the method comprising a step of using a first primer homologous to at least a portion downstream from the 5' end of a specified nucleotide sequence of the RNA and a second primer complementary to at least a portion upstream from the 3' end of the specified nucleotide sequence (where at least one of the first and second primers has a promoter sequence at the 5' end) to produce double-stranded DNA containing the promoter sequence and the specified nucleotide sequence downstream from the promoter sequence, a step of using the double-stranded DNA as template to produce an RNA transcript, a step of using the RNA transcript in turn as template for DNA synthesis to produce the double-stranded DNA, a step of nucleic acid amplification in which the aforementioned steps are repeated under conditions that simultaneously promote each of the steps, and a step of assaying the amount of the RNA transcript.

A sample according to the present invention consists of nucleic acid extracted by a known method from a specimen such as blood, serum, plasma, tissue or lavage suspected of containing cancer cells.

A specified nucleotide sequence according to the present invention consists of at least a partial sequence of hnRNP B1 mRNA or the sequence complementary thereto, and it is the sequence of the region defined between the first primer and second primer. According to the present invention, the RNA transcript from the specified nucleotide sequence is amplified. When the promoter sequence is added to the first primer, the hnRNP B1 mRNA is preferably cleaved at the 5'-end of the specific nucleic acid sequence before serving as the template for cDNA synthesis. The cleavage method is not particularly restricted, but it is preferably a method using an enzyme with RNase H activity to cleave the RNA portion of an RNA-DNA hybrid formed by adding an oligonucleotide (a cleavage oligonucleotide) having a sequence complementary to the region overlapping and adjacent to the 5'-end of the specific nucleotide sequence of hnRNP B1 mRNA, and preferably the cleavage oligonucleotide used has its 3'-terminal -OH appropriately modified, for example, aminated, to prevent extension reaction.

A target nucleic acid according to the present invention has a region in the specific base sequence that is not homologous or complementary to the first and second primers, while having a sequence that allows complementary binding with the intercalating fluorescent dye-labeled nucleic acid probe. Thus, the intercalating fluorescent dye-labeled nucleic acid probe is a sequence complementary to a portion of the specific nucleotide sequence according to the invention. According to one mode of the present invention, therefore, when the specified nucleotide sequence is a sequence homologous to hnRNP B1 mRNA, the intercalating fluorescent dye-labeled nucleic acid probe has a sequence containing at least 15 contiguous bases of the sequence listed as SEQ ID NO: 3, and when the specified nucleotide sequence is a sequence complementary to hnRNP B1 mRNA, the intercalating fluorescent dye-labeled nucleic acid probe has a sequence containing at least 15 contiguous bases of a sequence complementary to the sequence listed as SEQ ID NO: 3.

At least one of the first and second primers according to the present invention has a promoter sequence at the 5'-end, and are oligonucleotides with sufficient complementarity to a complementary sequence for hnRNP B1 mRNA in the case of the first primer and to hnRNP B1 mRNA in the case of the second primer. "Sufficient complementarity" means that complementary binding can occur with the specified nucleotide sequence or the sequence complementary to that sequence, under the reaction conditions (reaction temperature and composition of salts, etc.) for the nucleic acid amplification step of the invention. Such reaction conditions may be, for example, hybridization conditions at 43°C in the presence of 60 mM Tris, 17 mM magnesium chloride, 100 mM potassium chloride and 1 mM DTT.

In order to provide sufficient complementarity to a complementary sequence for hnRNP B1 mRNA for the first primer, to hnRNP B1 mRNA for the second primer and to the target nucleic acid for the intercalating fluorescent dye-labeled nucleic acid probe, the first primer preferably includes at least 15 and more preferably at least 20 contiguous bases of the sequence listed as SEQ ID NO: 1, the second primer preferably includes at least 15 and more preferably at least 20 contiguous bases of the sequence listed as SEQ ID NO: 2, and the intercalating fluorescent dye-labeled nucleic acid probe preferably includes at least 15 and more preferably at least 20 contiguous bases of the sequence listed as SEQ ID NO: 3 or the sequence complementary to that sequence.

Alternatively, the first primer, second primer and intercalating fluorescent dye-labeled nucleic acid probe may each have a nucleotide sequence that hybridizes with the complementary strand having the sequence listed as SEQ ID NOS: 1, 2 or 3, respectively, under highly stringent conditions, for example, under the aforementioned reaction conditions for the nucleic acid amplification step of the invention.

Since the first primer is designed based on sequence included in hnRNP B1 mRNA that is lacking in hnRNP A2 mRNA, it can be used for specific assay of hnRNP B1 mRNA alone.

The promoter sequence used for the present invention is a sequence to which RNA polymerase binds to initiate transcription, and specific sequences corresponding to different RNA polymerases are known. There are no particular restrictions on the RNA polymerase, but common ones such as T7 phage RNA polymerase, T3 phage RNA polymerase and SP6 phage RNA polymerase are preferred, and their corresponding promoter sequences may be used.

The method for assaying hnRNP B1 mRNA according to the present invention requires certain enzymes (an enzyme having RNA-dependent DNA polymerase activity for single-stranded RNA template (reverse transcriptase), an enzyme having RNase H activity, an enzyme having DNA-dependent DNA polymerase activity for single-stranded DNA template, and an enzyme having RNA polymerase activity). Any of these enzymes may be enzymes having different activities, or a plurality of enzymes having each activity may be used. For example, an enzyme having RNA polymerase activity may be added to a reverse transcriptase having RNA-dependent DNA polymerase activity for single-stranded RNA template, RNase H activity and DNA-dependent DNA polymerase activity for single-stranded DNA template, or if necessary an enzyme with RNase H activity may be further added as a supplement. Preferred as the reverse transcriptase are AMV reverse transcriptase, M-MLV reverse transcriptase and their derivatives, from the standpoint of flexible utility.

When the reverse transcription reaction is carried out in the presence of the first primer, the second primer and hnRNP B1 mRNA, the second primer binds to the specified base sequence of hnRNP B1 mRNA and carries out cDNA synthesis with the enzyme having RNA-dependent DNA polymerase activity. The RNA portion of the obtained RNA-DNA hybrid is degraded by the enzyme having RNase H activity, and dissociates so that the first primer may bind to the cDNA.

Next, double-stranded DNA derived from the specified base sequence and containing the promoter sequence at the 5'-end is produced by the enzyme having DNA-dependent DNA polymerase activity. The double-stranded DNA contains the specified base sequence downstream from the promoter sequence, and an RNA transcript derived from the specified base sequence is produced by the enzyme having RNA polymerase activity. The RNA transcript serves as template for the double-stranded DNA synthesis by the first and second primers, so that a series of reactions occur as a chain reaction and result in amplification of the RNA transcript.

In order to promote the chain reaction, it is of course necessary to add at least buffer solution, magnesium salt, potassium salt, nucleoside triphosphates and ribonucleoside triphosphates as known elements essential for each of the enzymes. In addition, additives such as dimethylsulfoxide (DMSO), dithiothreitol (DTT), bovine serum albumin (BSA), sugars and the like may be added to control the reaction efficiency.

For example, when using AMV reverse transcriptase and T7 RNA polymerase, the reaction temperature is preferably set in a range of 35°C-65°C, and most preferably it is set in a range of 40°C-44°C. The RNA amplification step proceeds isothermally, and the reaction temperature may be set to any desired temperature at which the reverse transcriptase and RNA polymerase exhibit their activity.

The amount of amplified RNA transcript can be measured by a known nucleic acid assay method. As such assay methods, there may be used methods employing electrophoresis or liquid chromatography, or hybridization methods employing nucleic acid probes labeled with detectable labels. But these procedures involve multiple steps, and because the amplification product is removed out of the system for analysis, there is a high risk of escape of the amplification product into the environment as a cause of secondary contamination. To overcome these problems it is preferred to use a nucleic acid probe designed so that its fluorescent property changes upon complementary binding to the target nucleic acid. As a more preferred method, there may be mentioned a method wherein the nucleic acid amplification step is carried out in the presence of a nucleic acid probe which is labeled with an intercalating fluorescent dye and is designed so that when it forms a complementary double strand with the target nucleic acid, the intercalating fluorescent dye moiety undergoes a change in fluorescent property by intercalating into the complementary double strand, and the change in fluorescent property is measured (see Japanese Unexamined Patent Publication (Kokai) No. 2000-14400 and Ishiguro, T., et al., (2003) Anal. Biochem., 314, 77-86).

There are no particular restrictions on the intercalating fluorescent dye, and there may be used common dyes such as oxazole yellow, thiazole orange, ethidium bromide and their derivatives. The change in fluorescent property may be a change in fluorescent intensity. For example, it is known that in the case of oxazole yellow, intercalation into double-stranded DNA causes a notable increase in fluorescence at 510 nm (excitation wavelength of 490 nm). The intercalating fluorescent dye-labeled nucleic acid probe is an oligonucleotide with sufficient complementarity to the RNA transcript, and it has a structure with the intercalating fluorescent dye bonded to an end, a phosphate diester moiety or a base moiety via an appropriate linker, and with the 3'-terminal -OH group suitably modified to prevent extension from the 3'-terminal -OH (see Japanese Unexamined Patent Publication (Kokai) No. 8-211050).

Labeling of the oligonucleotide with the intercalating fluorescent dye may be accomplished by introducing a functional group into the oligonucleotide by a known method and bonding the intercalating fluorescent dye thereto (see Japanese Unexamined Patent Publication (Kokai) No. 2001-13147 and Ishiguro, T., et al., (1996) Nucleic Acids Res., 24, 4992-4997). The method of introducing the functional group may employ the commonly used Label-ON Reagents (Clontech Laboratories, Inc.).

As one mode of the present invention, there are added to the sample an assay reagent containing at least a first primer (including at least 15 contiguous bases of the sequence listed as SEQ ID NO: 1) having the T7 promoter sequence at the 5' end, a second primer (including at least 15 contiguous bases of the sequence listed as SEQ ID NO: 2), an intercalating fluorescent dye-labeled nucleic acid probe (including at least 15 contiguous bases of the sequence listed as SEQ ID NO: 3), a cleavage oligonucleotide (including at least 15 bases of a sequence selected from among SEQ ID NOS: 18-21, and having a sequence complementary to the region overlapping and adjacent to the 5'-end of the specified base sequence), AMV reverse transcriptase, T7 RNA polymerase, a buffer, a magnesium salt, a potassium salt, nucleoside triphosphates, ribonucleoside triphosphates and dimethylsulfoxide (DMSO), and the reaction is carried out at a constant reaction temperature of 35-65°C (preferably 40-44°C) while periodically measuring the fluorescent intensity of the reaction solution. In this case, the fluorescent intensity results as an increase curve from the initial RNA amount, and therefore by drawing a calibration curve using standard RNA of known concentration it is possible to quantify the initial amount of RNA in an unknown sample. Moreover, since the fluorescent intensity is periodically measured, the measurement may be concluded at any desired point at which a significant increase in fluorescence is detected, and measurement results can be obtained usually within an hour, and within 30 minutes with an optimal system.

It is especially notable that all of the test materials in the assay reagent can be included in the same vessel. That is, the simple procedure of dispensing prescribed amounts of test materials into a single vessel will allow automatic amplification and detection of hnRNP B1 mRNA to be conducted thereafter. The vessel may be constructed of, for example, a partially transparent material to allow external measurement of the signal emitted by the fluorescent dye, and a vessel that can be sealed after dispensing the test materials is particularly preferred to prevent contamination.

The RNA amplification and assay method according to the modes described above can be carried out in a single-stage and isothermal manner, and it is therefore more convenient than RT-PCR and is suitable for automation. However, because the reaction is carried out at a relatively low constant temperature of 35-65°C without denaturation and annealing steps as in RT-PCR, it is prone to be affected by non-specific amplification products such as primer dimers or by the higher order structure of the RNA to be assayed, and therefore a much more elaborate design is necessary to construct the assay system compared to RT-PCR, for which reason it had not been previously possible to assay hnRNP B1 mRNA in a rapid, convenient, isothermal and single-stage manner using the aforementioned RNA amplification and assay method. According to the present invention it possible for the first time to assay hnRNP B1 mRNA with high specificity and high sensitivity in a rapid, convenient, isothermal and single-stage manner.

The present invention can be applied as an index for early diagnosis of lung cancer and other squamous carcinoma, for treatment effect monitoring of chemotherapy and the like, for diagnosis of micrometastasis, for predicting prognosis and for determining treatment course.

### Examples

The present invention will now be further explained by examples, with the understanding that the invention is not limited by the examples.

### Example 1

hnRNP B1 RNA was prepared by *in vitro* transcription of double-stranded DNA comprising hnRNP B1 cDNA (including bases 157-1249, with numbers according to National Center Biotechnology Information accession No. NM_031243) downstream from the SP6 phage RNA polymerase promoter, followed by complete digestion of the double-stranded DNA by DNaseI treatment and purification of the RNA. The RNA was quantitated by measuring the absorbance at 260 nm.

The following examples deal with RNA as the object of measurement, but they are entirely applicable for assay of hnRNP B1 mRNA as the object of measurement according to the invention.

### Example 2

Oligonucleotide probes labeled with an intercalating fluorescent dye were prepared. Amino groups were introduced at the positions of the 13th bases from the 5'-ends of the sequences listed as SEQ ID NOS: 16 and 17 (A in SEQ ID NO: 16, T in SEQ ID NO: 17) using Label-ON Reagents (Clontech), and the 3'-ends were labeled with biotin. Oxazole yellow was bonded to the amino groups by the method described in Ishiguro et al. (ibid, 1996) (Fig. 1B). Also, oxazole yellow was bonded via a linker to the phosphate diester moiety between the 12th G and 13th A from the 5'-end of the sequence listed as SEQ ID NO: 16 by the method described in Ishiguro et al. (ibid, 1996), to prepare an oxazole yellow-labeled nucleic acid probe (Fig. 1A).

### Example 3

The method of the invention was used for detection of different initial numbers of copies of hnRNP B1 RNA.
(1) The aforementioned hnRNP B1 RNA (including base numbers 157-1249) was diluted to 25, 50, 100 and 1000 copies/5 µl using an RNA diluent (10 mM Tris/HCl (pH 8.0), 1 mM EDTA, 0.25 U/µl ribonuclease inhibitor, 5 mM DTT) for use as RNA samples. The RNA diluent was used as the negative standard (0 copies).
(2) After dispensing 20 µl of reaction mixture with the following composition into a 0.5 ml-volume PCR tube (Gene Amp Thin-Walled Reaction Tubes, Perkin-Elmer), 5 µl of the RNA sample was added.
   Reaction mixture composition: The final concentrations after addition to the enzyme solution (30 µl) were as follows.
   60 mM Tris/HCl (pH 8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO: 7): The first primer had the T7 polymerase promoter sequence (SEQ ID NO: 22) added to the 5'-end of the nucleotide sequence listed as this SEQ ID NO.
   1 µM second primer (SEQ ID NO: 14)
   25 nM intercalating fluorescent dye-labeled nucleic acid probe (SEQ ID NO: 16): The nucleic acid probe was prepared using Label-ON Reagents in Example 2
   0.16 µM cleavage oligonucleotide (SEQ ID NO: 20): The 3'-OH group of the oligonucleotide was modified with an amino group
   6 U/30 µl ribonuclease inhibitor (Takara Bio, Inc.) 13% DMSO.
(3) The reaction mixture was kept at 43°C for 5 minutes, and then 5 µl of enzyme solution with the following composition, kept at 43°C for 2 minutes, was added.
   Enzyme solution composition: Final concentrations at time of reaction (in 30 µl)
   2% sorbitol
   8 U/30 µl AMV reverse transcriptase (Takara Bio, Inc.)
   142 U/30 µl T7 RNA polymerase (GIBCO)
   3.6 µg/30 µl bovine serum albumin
(4) Next, a fluorescent spectrophotometer equipped with a heat controlling function and allowing direct measurement of the PCR tube was used for reaction at 43°C while periodically measuring the fluorescent intensity of the reaction mixture (excitation wavelength: 470 nm, fluorescent wavelength: 520 nm).

The time-related change in the fluorescent intensity ratio of the reaction mixture (fluorescent intensity value at prescribed time ÷ background fluorescent intensity value) is shown in Fig. 2, where the point of enzyme addition is defined as 0 minutes.

The results in Fig. 2 show a fluorescent profile dependent on the initial concentration of hnRNP B1 RNA, whereby 25 copies/test of hnRNP B1 RNA could be detected within 20 minutes. This indicates that hnRNP B1 RNA can be assayed at high sensitivity and high speed by the method of the present invention.

### Example 4

hnRNP B1 RNA was assayed by the method of the present invention using different combinations of first primer, second primer, intercalating fluorescent dye-labeled nucleic acid probe and cleavage oligonucleotide.
(1) The aforementioned hnRNP B1 RNA (including base numbers 157-1249) was diluted to 10³ or 10² copies/5 µl using an RNA diluent (10 mM Tris/HCl (pH 8.0), 1 mM EDTA, 0.25 U/µl ribonuclease inhibitor, 5.0 mM DTT) to prepare RNA samples.
(2) After dispensing 20 µl of reaction mixture with the following composition into a 0.5 ml-volume PCR tube (GeneAmp Thin-Walled Reaction Tubes, Perkin-Elmer), 5 µl of the RNA sample was added.
   Reaction mixture composition: Concentrations are final concentrations (in 30 µl) after addition of enzyme solution.
   - 60 mM: Tris/HCl (pH 8.6)
   - 17 mM: magnesium chloride
   - 100 mM: potassium chloride
   - 1 mM: DTT
   - 0.25 mM: each of dATP, dCTP, dGTP, dTTP
   - 3 mM: each of ATP, CTP, UTP
   - 2.25 mM: GTP
   - 3.6 mM: ITP
   1 µM first primer (SEQ ID NOS. listed in Tables 1 and 2): The first primer had the T7 polymerase promoter sequence (SEQ ID NO: 22) added to the 5'-end of the nucleotide sequence listed as the respective SEQ ID NO.
   1 µM second primer (SEQ ID NOS. listed in Tables 1 and 2)
   25 nM intercalating fluorescent dye-labeled nucleic acid probe (SEQ ID NOS listed in Tables 1 and 2): The nucleic acid probes were prepared using the Label-ON Reagents of Example 2
   0.16 µM cleavage oligonucleotide (SEQ ID NOS. listed in Tables 1 and 2): The 3'-OH group of the oligonucleotide was modified with an amino group.
   6U/30 µl ribonuclease inhibitor (Takara Bio, Inc.) 13% DMSO
(3) The reaction mixture was kept at 43°C for 5 minutes, and then 5 µl of enzyme solution with the following composition, kept at 43°C for 2 minutes, was added.
   Enzyme solution composition: Final concentrations at time of reaction (in 30 µl)
   2% sorbitol
   8 U/30 µl AMV reverse transcriptase (Takara Bio, Inc.)
   142 U/30 µl T7 RNA polymerase (GIBCO)
   3.6 µg/30 µl bovine serum albumin
(4) Next, a fluorescent spectrophotometer equipped with a heat regulating function and allowing direct measurement of the PCR tube was used for reaction at 43°C while periodically measuring the fluorescent intensity of the reaction mixture (excitation wavelength: 470 nm, fluorescent wavelength: 520 nm).

Tables 1 and 2 show the results, where (+) indicates that the fluorescent intensity ratio of the reaction mixture exceeded 1.2, and the time at that point is listed as the detection time, with the point of enzyme addition defined as 0 minutes.

When using the combinations of first primer, second primer, intercalating fluorescent dye-labeled nucleic acid probe and cleavage oligonucleotide listed in Tables 1 and 2, 10² or 10³ copies/test of hnRNP B1 RNA were detected within 30 minutes.

Specifically, hnRNP B1 RNA could be rapidly detected when using a combination consisting of a sequence selected from among SEQ ID NOS: 4-8 as the first primer, a sequence selected from among SEQ ID NOS: 9-15 as the second primer, the sequence of SEQ ID NO: 16 or 17 as the intercalating fluorescent dye-labeled nucleic acid probe and a sequence selected from among SEQ ID NOS: 18-21 as the cleavage oligonucleotide. SEQ ID NOS: 4-8 are partial sequences of SEQ ID NO: 1, SEQ ID NOS: 9-15 are partial sequences of SEQ ID NO: 2, and SEQ ID NOS: 16 and 17 are partial sequences of SEQ ID NO: 3. This test indicated that the RNA amplification and assay method using the first primers, second primers and intercalating fluorescent dye-labeled nucleic acid probes of the invention allow rapid detection of hnRNP B1 RNA.

Table 1 shows the results of assays using different oligonucleotide combinations

**Table 1**

| Oligonucleotide combination | | | | Assay results | |
|---|---|---|---|---|---|
| First primer (SEQ ID NO) | Second primer (SEQ ID NO) | Intercalating fluorescent dye-labeled nucleic acid probe (SEQ ID NO) | Cleavage Oligonucleotide (SEQ ID NO) | Detection time (min) | Judgment |
| 5 | 10 | 16 | 19 | 11.5 | + |
| 5 | 10 | 17 | 19 | 24.1 | + |
| 5 | 12 | 16 | 19 | 11.6 | + |
| 8 | 12 | 16 | 21 | 16.3 | + |

These oligonucleotide combinations were used for RNA amplification and fluorescence assay using 10³ copies/test of hnRNP B1 RNA as sample. Samples with a fluorescent intensity ratio exceeding 1.2 were indicated as (+), and the time at that point was recorded as the detection time.

Table 2 shows the results of assays using different oligonucleotide combinations

**Table 2**

| Oligonucleotide combination | | | | Assay results | |
|---|---|---|---|---|---|
| First primer (SEQ ID NO) | Second primer (SEQ ID NO) | Intercalating fluorescent dye-labeled nucleic acid probe (SEQ ID NO) | Cleavage Oligonucleotide (SEQ ID NO) | Detection time (min) | Judgment |
| 4 | 9 | 16 | 18 | 17.1 | + |
| 4 | 11 | 16 | 18 | 18.8 | + |
| 4 | 12 | 16 | 18 | 25.0 | + |
| 4 | 13 | 16 | 18 | 15.7 | + |
| 4 | 14 | 16 | 18 | 16.4 | + |
| 6 | 14 | 16 | 19 | 16.4 | + |
| 7 | 11 | 16 | 20 | 20.1 | + |
| 7 | 12 | 16 | 20 | 16.5 | + |
| 7 | 13 | 16 | 20 | 21.2 | + |
| 7 | 14 | 16 | 20 | 15.1 | + |
| 7 | 15 | 16 | 20 | 13.2 | + |

These oligonucleotide combinations were used for RNA amplification and fluorescence assay using 10² copies/test of hnRNP B1 RNA as sample. Samples with a fluorescent intensity ratio exceeding 1.2 were indicated as (+), and the time at that point was recorded as the detection time.

### Example 5

The method of the invention was used for quantitation of hnRNP B1 RNA.
(1) The aforementioned hnRNP B1 RNA (including base numbers 157-1249) was diluted to 10², 10³, 10⁴, 10⁵ and 10⁶ copies/5 µl using an RNA diluent (10 mM Tris/HCl (pH 8.0), 1 mM EDTA, 0.25 U/µl ribonuclease inhibitor, 5.0 mM DTT) and used for calibration curve standard RNA. The diluent was used as the negative standard (NEG). Samples L (10³ copies/5 µl), M (10⁴ copies/5 µl) and H (10⁵ copies/5 µl) were prepared in a similar manner.
(2) After dispensing 20 µl of reaction mixture with the following composition into a 0.5 ml-volume PCR tube (Gene Amp Thin-Walled Reaction Tubes, Perkin-Elmer), 5 µl each of the standard RNA and sample was added. Reaction mixture composition: Concentrations are final concentrations (in 30 µl) after addition of enzyme solution.
   60 mM Tris/HCl (pH 8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO: 7): The first primer had the T7 polymerase promoter sequence (SEQ ID NO: 22) added to the 5'-end of the nucleotide sequence listed as this SEQ ID NO.
   1 µM second primer (SEQ ID NO: 14)
   25 nM intercalating fluorescent dye-labeled nucleic acid probe (SEQ ID NO: 16): The nucleic acid probe is one having oxazole yellow bonded to the phosphate diester via a linker as described in Example 2
   0.16 µM cleavage oligonucleotide (SEQ ID NO: 20): The 3'-OH group of the oligonucleotide was modified with an amino group
   6 U/30 µl ribonuclease inhibitor (Takara Bio, Inc.) 13% DMSO
(3) The reaction mixture was kept at 43°C for 5 minutes, and then 5 µl of an enzyme solution with the following composition, kept at 43°C for 2 minutes, was added.
   Enzyme solution composition: Final concentrations at time of reaction (in 30 µl)
   2% sorbitol
   8 U/30 µl AMV reverse transcriptase (Takara Bio, Inc.)
   142 U/30 µl T7 RNA polymerase (GIBCO)
   3.6 µg/30 µl bovine serum albumin
(4) Next, a fluorescent spectrophotometer equipped with a heat controlling function and allowing direct measurement of the PCR tube was used for reaction at 43°C while periodically measuring the fluorescent intensity of the reaction mixture (excitation wavelength: 470 nm, fluorescent wavelength: 520 nm).

The time-related change in the fluorescent intensity ratio of the reaction mixture (fluorescent intensity value at prescribed time ÷ background fluorescent intensity value) is shown in Fig. 3, where the point of enzyme addition is defined as 0 minutes. With the detection time defined as the time at which the fluorescent intensity ratio reached 1.2 based on the results in Fig. 3, a calibration curve was drawn from the detection time and logarithm of initial number of copies, as shown in Fig. 4. Also, the numbers of copies of samples L, M and H were quantified from the calibration curve and the results are shown in Table 3.

In the results shown in Fig. 4, 10² copies/test were detected at approximately 12 minutes, and satisfactory linearity was obtained between detection time and log of initial number of copies. The quantitation results for samples L, M and H in Table 3 were also values corresponding to the amount of hnRNP B1 RNA added.

Thus, it was demonstrated that the method of the invention can quantify hnRNP B1 RNA in a rapid, highly sensitive and specific manner.

The results of hnRNP B1 RNA quantitation are shown in Table 3.

**Table 3**

| | Assay results | |
|---|---|---|
| Sample | Detection time (min) | No. of copies/test |
| L | 10.25 | 1.4 x 10³ |
| M | 9.23 | 1.1 x 10⁴ |
| H | 8.22 | 9.4 x 10⁴ |

Results of calculating number of copies based on detection time obtained from the fluorescent profile for the measurement results for samples L (10³ copies/5 µl), M (10⁴ copies/5 µl) and H (10⁵ copies/5 µl), and calibration curve of Fig. 4.

### Industrial Applicability

According to the present invention it has become possible to achieve highly sensitive assay of hnRNP B1 mRNA in an isothermal, single-stage, convenient and rapid manner. The invention is therefore suitable as an index for early diagnosis of lung cancer and other squamous carcinoma, and is useful for monitoring of the treatment effects of chemotherapy and the like, for predicting prognosis and for determining treatment course. Since the invention can be carried out in a single stage and in a sealed vessel, it is possible to minimize the risk of contamination of the environment by amplification product as a secondary contaminant.

Furthermore, since the method is also carried out in a single-stage, convenient and rapid manner, multiple specimens can be processed even by manual methods, and it is possible to minimize the number of procedures that may reduce reproducibility. In addition, since the RNA amplification method of the invention amplifies only RNA, it is possible to achieve precise amplification and assay of mRNA without a step of completely removing double-stranded DNA as is required in RT-PCR. In other words, the method of the invention is optimal for highly sensitive and rapid expression analysis. Moreover, because it can be carried out in an isothermal and single-stage manner, there is no need to provide a thermal cycling mechanism as in the PCR, and automation is thus facilitated.

## Claims

1. A method for assaying heterogeneous nuclear ribonucleoprotein B1 (hnRNP B1) mRNA present in a sample, the method comprising
(1) a step of using a first primer homologous to at least a portion downstream from the 5' end of a specified nucleotide sequence of the RNA and a second primer complementary to at least a portion upstream from the 3' end of the specified nucleotide sequence to produce double-stranded DNA containing a promoter sequence and the specified nucleotide sequence downstream from the promoter sequence, wherein at least one of said first and second primers has the promoter sequence at the 5' end,
(2) a step of using the double-stranded DNA as template to produce said RNA transcript,
(3) a step of using said RNA transcript in turn as template for DNA synthesis for amplification of the RNA transcript in a chain reaction, and
(4) a step of assaying the amount of the RNA transcript.

2. A method for assaying hnRNP B1 mRNA according to claim 1, **characterized in that** the assay of the amount of the RNA transcript is accomplished by measuring the change in the fluorescent property of a nucleic acid probe that is labeled with an intercalating dye and is designed so that when it forms a complementary double strand with the target nucleic acid, the intercalating fluorescent dye moiety undergoes a change in fluorescent property by intercalating into the complementary double strand.

3. A method for assaying hnRNP B1 mRNA according to claim 1 or 2, **characterized in that** said first primer includes at least 15 contiguous bases of the sequence listed as SEQ ID NO: 1, and/or said second primer includes at least 15 contiguous bases of the sequence listed as SEQ ID NO: 2.

4. A method for assaying hnRNP B1 mRNA according to claim 2, **characterized in that** said first primer comprises at least 15 contiguous bases of the sequence listed as SEQ ID NO: 1 and said second primer comprises at least 15 contiguous bases of the sequence listed as SEQ ID NO: 2, at least one of the first and second primers contain a promoter sequence at the 5'-end, and when the first primer contains the promoter sequence, it comprises an intercalating fluorescent dye-labeled nucleic acid probe including at least 15 contiguous bases of the sequence listed as SEQ ID NO: 3, and when the second primer contains the promoter sequence, it comprises an intercalating fluorescent dye-labeled nucleic acid probe including at least 15 contiguous bases of the sequence complementary to the sequence listed as SEQ ID NO: 3.

5. An assay reagent for hnRNP B1 mRNA, **characterized by** comprising as constituent elements:
an oligonucleotide containing at least 15 contiguous bases of the sequence listed as SEQ ID NO: 1 as a first primer;
an oligonucleotide containing at least 15 contiguous bases of the sequence listed as SEQ ID NO: 2 as a second primer, wherein at least one of the first and second primers contain a promoter sequence at the 5'-end; and
when the first primer contains the promoter sequence, an intercalating fluorescent dye-labeled nucleic acid probe including at least 15 contiguous bases of the sequence listed as SEQ ID NO: 3, and when the second primer contains the promoter sequence, an intercalating fluorescent dye-labeled nucleic acid probe including at least 15 contiguous bases of the sequence complementary to the sequence listed as SEQ ID NO: 3.
